# EUROPEAN PATENT APPLICATION

(11) **EP 2 659 833 A2**
(43) Date of publication of application: **06.11.2013**
(21) Application number: 11853370.2
(22) Date of filing: 29.12.2011
(51) Int. Cl.: A61B 5/02, A61B 5/022

(54) **TWO-ARM BLOOD PRESSURE MEASUREMENT APPARATUS FOR AUTOMATICALLY MEASURING TWO-ARM BLOOD PRESSURES AT THE SAME TIME**

(30) Priority: 30.12.2010 KR 20100138631; 14.12.2011 KR 20110134175
(71) Applicant: Jawon Medical Co., Ltd, Gyeongsan-si, Gyeongsangbuk-do 712-838 (KR)
(72) Inventor: PARK, Won-Hee, Gyeongsan-si Gyeongsangbuk-do 712-838 (KR)
(74) Representative: Viering, Jentschura & Partner
(86) International application number: PCT/KR2011/010323
(87) International publication number: WO 2012/091497

(57) **Abstract**

Disclosed is an apparatus for measuring blood pressures of both arms, in which an examine simultaneously measures the blood pressures of both arms without getting the arms out of cuffs in the stable and safely posture. The apparatus includes a body (100) mounted on a mounting table such as a desk, a left cuff unit (110) placed at a left upper portion of the body (100), and a right cuff unit (210) placed at a right upper portion of the body (100). The left and right cuff units have the symmetric structure so that the blood pressure is measured at identical blood pressure measurement portions of the left and right arms of the examinee which are placed at internal space parts of the left and right cuff units. Front openings have an identical shape, rear openings have an identical shape, the internal space parts have an equal size, and bladders of the left and right cuff units have an equal volume such that the blood pressures are measured at the identical blood pressure measurement portions of the left and right arms of the examinee, which are placed in internal space parts of the left and right cuff units, respectively, under an identical condition. The CPU simultaneously drives the left and right cuff units to simultaneously measure the blood pressures of the left and right arms, if the left and right arms of the examinee are placed in the internal space parts of the left and right cuff units.

## Description

### [Technical Field]

The present invention relates to an apparatus for automatically and simultaneously measuring blood pressures of both arms. In particular, the present invention relates to an apparatus for automatically and simultaneously measuring blood pressures of both arms, which can measure the state related to the blood flow or the blood pressure state by measuring the difference between blood pressures of both arms of a human body.

### [Background Art]

One of important conditions for the proper treatment of the hypertension issued in modern society is to measure the blood pressure by using a reliable blood pressure measuring device.

It is generally known to those skilled in the art that the blood pressure can be most suitably measured when an examinee measures the blood pressure in a stable posture and a comfortable state without stress in order to exactly measure the blood pressure. Since the blood pressure varies in every heartbeat, the blood pressure must be measured several times in order to obtain reliable results.

In particular, most hypertensive patients are undergone treatment through a primary healthcare system. Accordingly, the exact measurement through the primary healthcare system is the first step to reduce a death rate caused by a cardiovascular disease.

Until now, when hypertension is diagnosed, the blood pressures of both arms of a patient are measured. If the difference between the blood pressures of the both arms is made, the blood pressures of the both arms of the patient are measured again, and a higher blood pressure is selected. In addition, a guideline to measure the blood pressure from the arm having the higher blood pressure is suggested when the patient measures the blood pressures thereafter. The slight difference between the blood pressures of both arms is made even in the case of a normal person. However, the great difference between the blood pressures of both arms indicates the peripheral artery disease, and increases the morbidity rate to warn cardiovascular disease and the death rate resulting from the cardiovascular disease.

The world health organization (WHO) indicates that the difference between the blood pressures of both arms in the range of 10 mmHg to 20 mmHg serves as the risk factor of the diseases of the circulatory system.

Japanese Unexamined Patent Publication No. 2000-342117 discloses an apparatus including a left-side blood pressure measuring device to measure the blood pressures of a left arm and a right-side blood pressure measuring device to measure the blood pressure of a right arm. According to the apparatus, when the blood pressure of the left arm is measured, a micro-switch installed in a left arm support member is pressed, so that the measuring of the blood pressure of the left arm is started. When the blood pressure of the right arm is measured, a micro-switch installed in a right arm support member is pressed, so that the measuring of the blood pressure of the right arm is started. Accordingly, an examinee cannot simultaneously measure the blood pressures of the left and right arms, so that the examinee cannot check the exact difference between the blood pressures of the left and right arms.

Japanese Unexamined Patent Publication No. 2010-119639 discloses an apparatus to automatically recognize the measurement of the blood pressure of a left arm when the left arm is inserted into a cuff unit, and automatically recognize the measurement of the blood pressure of a right arm when the right arm is inserted into the cuff unit. However, an examinee cannot simultaneously measure the blood pressures of the left and right arms under the same condition with one starting button.

Japanese Unexamined Patent Publication No. 2009-523512 discloses an apparatus to wind two cuffs around left and right arms of an examinee to measure the blood pressures of both of left and right arms of the examinee.

However, according to the blood pressure measuring apparatus, cuffs may be wound around different positions of both arms, and the left and right cuffs may be wound with different intensities. Accordingly, the blood pressures of the left and right arms cannot be measured at the same pressure.

In particular, since the apparatus disclosed in Japanese Unexamined Patent Publication No. 2009-523512 is designed to measure the blood pressures of a user based on press signals generated from two independent cuffs, the slight difference is made between the pressures of the left and right cuffs, and the amounts of air existing in the cuffs are slightly different from each other depending on the force of the cuffs wound around both arms. Therefore, the two cuffs require different times until the cuffs are pumped to a preset pressure. Accordingly, the measurements of the blood pressures of both arms are simultaneously finished by adjusting the contraction rate of the cuffs during the measurement of the blood pressure using two independently-controllable expansion valves. Therefore, times spent to measure the blood pressures of the both arms are actually different from each other.

The present invention has been developed by taking the problems of the above blood pressure measuring apparatus into consideration. According to the present invention, left and right cuff units have the same structure so that the blood pressures of left and right arms are simultaneously measured at the same position and the same pressure when the blood pressures of the left and right arms are measured. Since the left and right cuff units have an equal size in the same structure, the left and right cuff units have an equal internal space size. In addition, corresponding parts in the cells of the left and right cuff units have the same height. In addition, bladders provided in the cells of the left and right cuff units have an equal size and an equal volume.

Therefore, according to the present invention, an examinee can simultaneously measure the blood pressures of left and right arms at the same pressure while maintaining the measurement portions of the left and right arms at the same position and the same height. As soon as the blood pressures of the left and right arms of the examinee are finished, the blood pressure measurement values of the left and right arms of the examinee can be displayed in terms of the difference between the blood pressures of the left and right arms on a result screen image.

### [Disclosure]

### [Technical Problem]

In the following description of the present invention, the same condition refers to that the measurement portions of both arms have an equal height, and an equal pressure is applied to the measurement portions. In addition, the term "simultaneously" refers to that the blood pressures of both arms are measured at the same time by using one starting button.

An object of the present invention is to provide an apparatus for automatically blood pressures, which can simultaneously measure the blood pressures of both arms of an examinee under the same condition, and a display for displaying the difference between the blood pressures.

Another object of the present invention is to provide an apparatus for automatically measuring blood pressures and a display to display the difference between the blood pressures, in which an examine can simultaneously and safely measure the blood pressures without getting the arms out of cuffs at the constant posture after placing both arms into the cuffs under the same condition.

Still another object of the present invention is to provide an apparatus for measuring blood pressures of both arms, which can be easily and simply driven by even the old and infant.

Still yet another object of the present invention is to provide an apparatus for automatically measuring blood pressures, which can simultaneously measure the blood pressures of both arms, and arbitrarily select one of left and right arms to simply measure the blood pressure of the selected arm, and a display to display the difference between the blood pressures.

### [Technical Solution]

In order to accomplish the above object, there is provided an apparatus for measuring blood pressures of both arms, which includes left and right cuff units receiving arms of an examinee therein and including bladders automatically wound around the arms in an operation, and a control unit (CPU) controlling the left and right cuff units.

The left and right cuff units are symmetric to each other such that the blood pressures are measured at identical blood pressure measurement portions of the left and right arms of the examinee placed in internal space parts of the left and right cuff units, respectively, and front openings have an identical shape, rear openings have an identical shape, the internal space parts have an equal size, and bladders of the left and right cuff units have an equal volume such that the blood pressures are measured at the identical blood pressure measurement portions of the left and right arms of the examinee, which are placed in internal space parts of the left and right cuff units, respectively, under an identical condition.

The control unit simultaneously drives the left and right cuff units to simultaneously measure the blood pressures of the left and right arms, if the left and right arms of the examinee are placed in the internal space parts of the left and right cuff units.

The apparatus includes the bladders of the left and right cuff units surrounding the identical blood pressure measurement portions of the left and right arms of the examinee.

The apparatus includes the bladders of the left and right cuff units which are are in a plane-symmetric to each other to surround arteries of the both arms.

### [Advantageous Effects]

As described above, the apparatus according to the present invention has a symmetric structure so that the blood pressures are measured at the identical blood pressure measurement portions of the left and right arms when the blood pressures of both arms are simultaneously and automatically measured. The front openings have the identical shape, the rear openings have the identical shape, the internal space parts have the equal size, and the bladders of the left and right cuff units have the equal volume such that the blood pressures are measured at the identical blood pressure measurement portions of the left and right arms of the examinee, which are placed in internal space parts of the left and right cuff units, respectively, under an identical condition.

The control unit simultaneously drives the left and right cuff units to simultaneously measure the blood pressures of the left and right arms, if the left and right arms of the examinee are placed in the internal space parts of the left and right cuff units. As soon as the measurement of the blood pressures is finished, the control unit displays the blood pressures of both arms and the difference between the blood pressures of the both arms.

### [Description of Drawings]

FIG. 1 is a perspective view showing an apparatus for measuring blood pressures according to the present invention.
FIG. 2 is a plan view showing the apparatus for measuring the blood pressures according to the present invention.
FIG. 3 is a partially cut perspective view showing left and right cuff units of FIG. 1.
FIG. 4 is a block diagram showing the circuit configuration of the apparatus for measuring the blood pressures according to the present invention.
FIG. 5 is a flowchart to explain the operation of the apparatus for measuring the blood pressures of both arms according to the present invention.
FIG. 6 is a flowchart showing the subroutine of the operation to measure the blood pressures of left and right arms in FIG. 5.
FIG. 7 is a flowchart showing the subroutine of the operation to measure the blood pressures of a left arm in FIG. 5.
FIG. 8 is a flowchart showing the subroutine of the operation to measure the blood pressures of a right arm in FIG. 5.
FIG. 9 is a flowchart to explain the operation of an emergency switch in the apparatus for measuring the blood pressures of both arms according to the present invention.
FIG. 10 is a perspective view showing an apparatus for measuring blood pressures of both arms according to another embodiment of the present invention.
FIG. 11 is a view showing starting and emergency switches according to the present invention.
FIG. 12 is a view showing the measurement result of the blood pressures of the left and right arms displayed on a screen image in terms of the maximum blood pressure, the minimum blood pressure, and the difference between the maximum blood pressure and the minimum blood pressure.

### [Best Mode]

### [Mode for Invention]

Hereinafter, an apparatus for measuring blood pressures of both arms according to the present invention will be described in detail with reference to accompanying drawings.

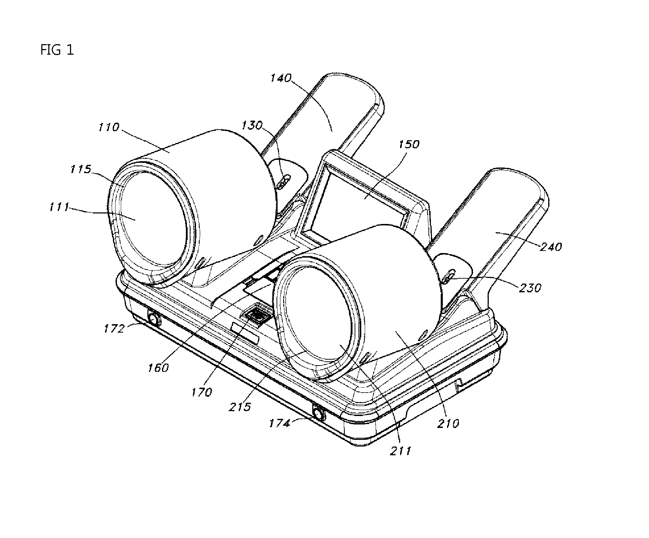

FIG. 1 is a perspective view showing an apparatus for measuring the blood pressures of both arms according to the present invention.

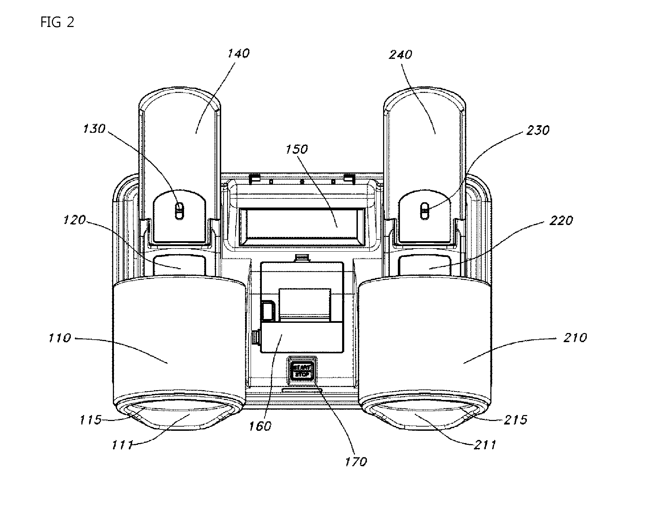

FIG. 2 is a plan view showing the apparatus for measuring the blood pressures of both arms of the examinee according to the present invention.

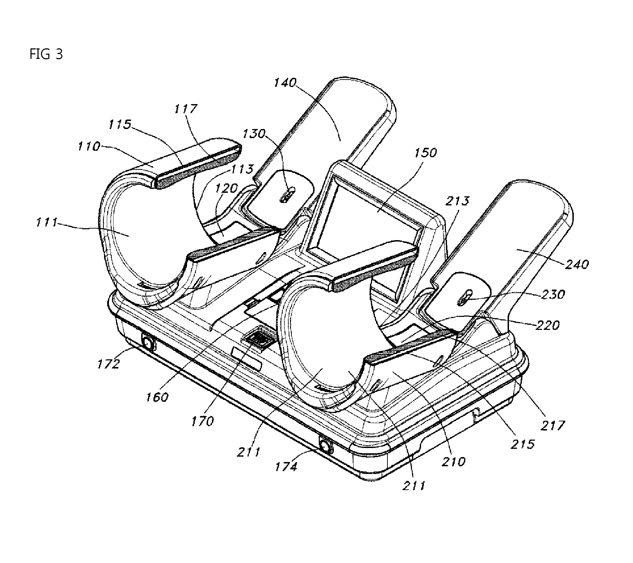

FIG. 3 is a partially cut perspective view showing left and right cuff units of FIG. 1.

Referring to FIGS. 1 to 3, an apparatus for automatically measuring blood pressures of both arms according to the embodiment of the present invention includes a body 100 mounted on a mounting table such as a desk, a left cuff unit 110 placed at a left upper portion of the body 100, and a right cuff unit 210 placed at a right upper portion of the body 100.

The left and right cuff units 110 and 210 include cells 115 and 215 having a substantially cylindrical shape and bladders 117 and 217 received in the cells 115 and 215. The cells 115 and 215 are provided therein with space parts extending from front openings 111 and 211 so that left and right arms of an examinee pass through space parts. The space parts are provided at rear portions thereof with rear openings 113 and 213 to allow the left and right arms introduced into the space parts to get out thereof.

Each of the bladders 117 and 217 is surrounded by fabric and received in related inner peripheral portions of the cells 115 and 215, respectively. The cells 115 and 215 have one surfaces surrounding the left and right arms of the examinee provided in the space parts of the cells 115 and 215 (see FIG. 3).

In the left and right cuff units 110 and 210, the cells 115 and 215 have an equal size and the same shape so that the blood pressures of the left and right arms of the examinee are measured under the same condition. In addition, the bladders 117 and 217 received in the inner peripheral portions of the cells 115 and 215, respectively, have the same shape and an equal volume. Accordingly, when the equal amount of air is injected into the bladders 117 and 217, the bladders 117 and 217 surround the left and right arms of the examinee passing through the cells 115 and 215, respectively, at the same pressure.

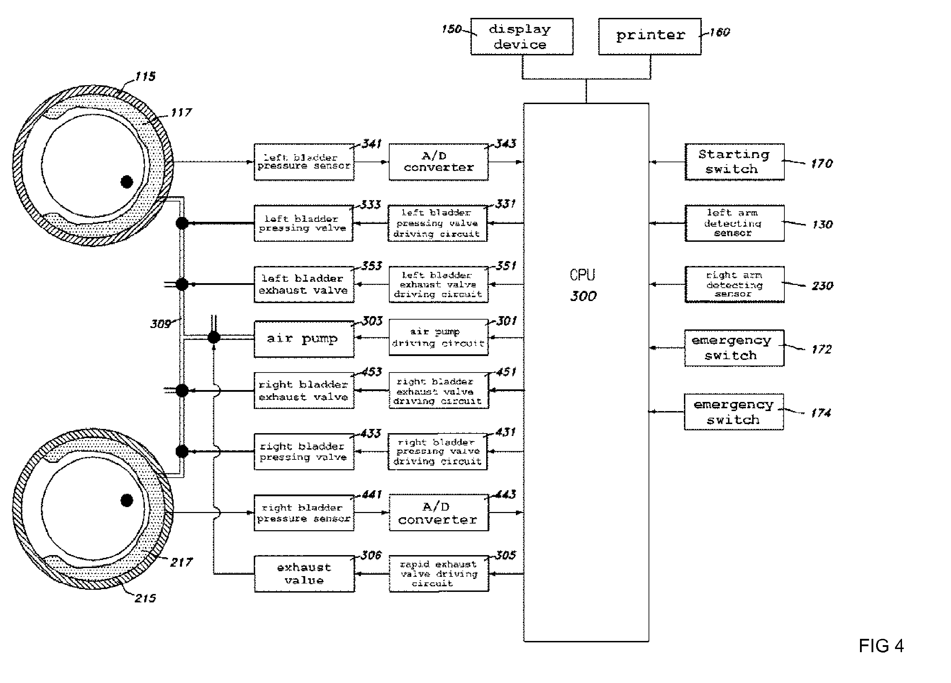

In addition, as shown in FIG. 4, the left and right bladders 117 and 217 are plane-symmetric to each other to measure the blood pressure at the same positions of arteries passing through the left and right arms symmetric to each other. This is because the arteries of the left and right arms are plane-symmetric to each other.

Accordingly, if an equal amount of air is injected into the bladders 117 and 217, the bladders 117 and 217 may surround the arteries of the left and right arms of the examinee at the equal pressure.

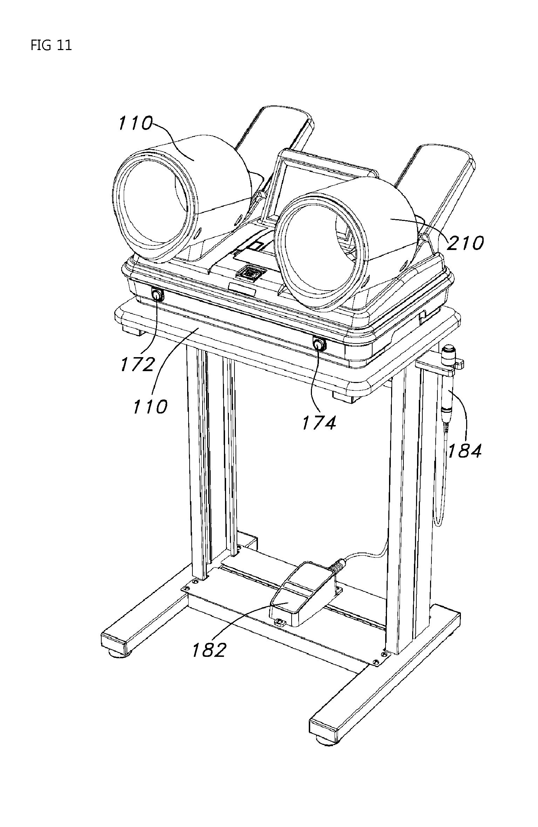

A display 150 is installed on a top surface of the body 100 between the left and right cuff units 110 and 210, and a printer 160 is installed in the front of the display 150. In addition, a starting switch 170 is exposed at the front of the top surface of the body 100. As shown in FIG. 11, the starting switch 170 may be provided in the form of a switch gripped by the hand of the examinee or the form of a switch pressed by the foot of the examinee, and may be connected to the body 100 for the use thereof. Emergency switches 172 and 174 are exposed at left and right sides of the front surface of the body 100. As shown in FIG. 11, the emergency switches 172 and 174 may be provided in the form of a switch gripped by the hand of the examinee or the form of a switch pressed by the foot of the examinee, and may be connected to the body 100 for the use thereof.

The rear openings 113 and 213 of the left and right cuff units 110 and 210 are provided at the rear portions thereof with elbow support members 120 and 220 at which the elbows of the examinee passing through the inner space parts of the left and right cuff units 110 and 210 are placed. Arm support members 140 and 240 are inclined upward from the elbow support members 120 and 220 to support left and right upper arms and left and right wrists of the examinee. In addition, the arm support members 140 and 240 are provided at the lower portions thereof with left and right arm detecting sensors 130 and 230 to detect the arms of the examinee.

The left and right arm detecting sensors 130 and 230 provided at the lower portions of the arm support members 140 and 240 detect the arms of the examinee placed on the arm support members 140 and 240 and diver the signals to a CPU.

When the examinee inserts lower arms and the wrists into the openings 111 and 211 of the left and right cuff units 110 and 210 to pass the lower arms and the wrists through the inner space parts of the left and right cuff units 110 and 210, places elbows on the elbow support members 120 and 220, and places the lower arms and the wrists on the arm support members 140 and 240 in an inclination direction along the arm support members 140 and 240, the measurement portions of left and right upper arms of the examinee are placed in the left and right cuff units 111 and 211 at the same height.

Accordingly, if an equal amount of air is injected into the left and right bladders 117 and 217, the left and right bladders 117 and 217 surround the measurement portions of the upper arms, which are placed at the same height, at an equal pressure.

The body 100 is provided therein with an electronic circuit device including an air pump to supply compressive air to the right and left bladders, a measurement circuit, and a central processing unit (CPU). The measurement results of the left and right cuff units may be displayed on the display 150 or output to the printer 160 under the control of the CPU.

FIG. 4 is a block diagram showing the structure of the apparatus for measuring blood pressures of both arms according to the present invention.

As shown in FIG. 4, the left bladder 117 constituting the left cuff unit 110 is connected to an air supply pipe 309 to inject air from an air pump 303. A left bladder pressure sensor 341 is attached to the left bladder 117 to detect the pressure of the left bladder 117. A left bladder pressing valve 333, a left bladder exhaust valve 353, and a rapid exhaust valve 306 are attached to the air supply pipe 309 to supply air to the left bladder 117 from the air pump 303. In addition, the rapid exhaust valve 306 is attached to the air supply pipe 309 to supply air to the left and right bladders 117 and 217.

The right bladder 217 constituting the right cuff unit 210 is connected to the air supply pipe 309 to inject air from the air pump 303. A right bladder pressure sensor 441 is attached to the right bladder 217 to detect the pressure of the right bladder 217. A right bladder pressing valve 433 and a right bladder exhaust valve 453 are attached to the air supply pipe 309 to supply air to the right bladder 217 from the air pump 303. In addition, the rapid exhaust valve 306 is attached to the air supply pipe 309 to supply air to the right bladders 217.

The pressure of the left bladder 117 is detected by the left bladder pressure sensor 341 and sent to the CPU 300 through a left A/D converter 343, and the pressure of the right bladder 217 is detected by the right bladder pressure sensor 441 and sent to the CPU 300 through a right A/D converter 443.

The air pump 303 is driven according to a driving signal of an air pump driving circuit 301 under the control of the CPU 300, and the rapid exhaust valve 306 attached to the air supply pipe 309 connected to the left and right bladders 117 and 217 is driven according to a driving signal of a rapid exhaust valve driving circuit 305 under the control of the CPU 300.

The CPU 300 receives pressure signals of the left and right bladders 117 and 217 from the left and right A/D converters 343 and 443 and transmits the driving signal of the air pump 303 to the air pump driving circuit 301.

In addition, the CPU 300 monitors signals of the left and right arm detecting sensors 130 and 230, the starting switch 170, and the emergency switches 172 and 174.

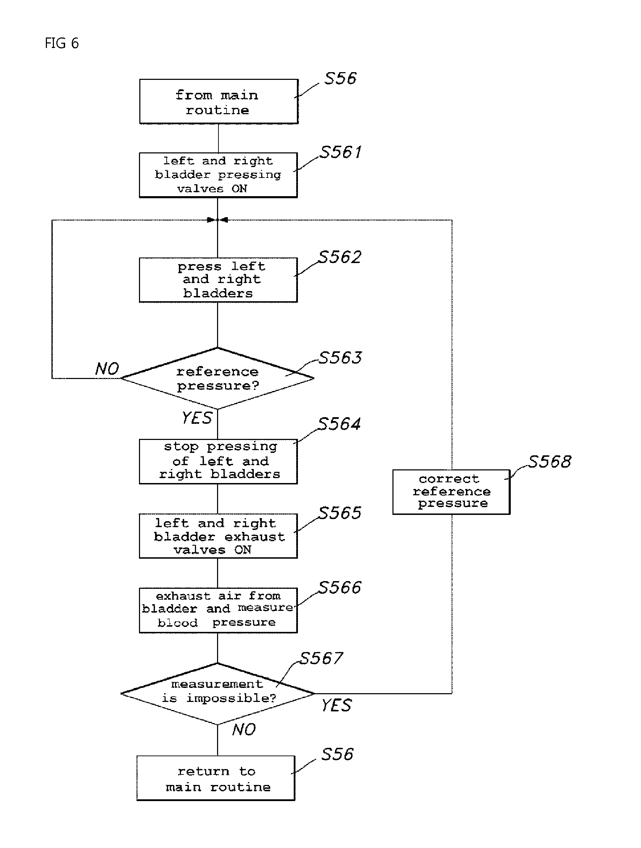

FIG. 6 is a flowchart showing the main routine of the operation of the apparatus 100 for measuring blood pressures of both arms according to the present invention.

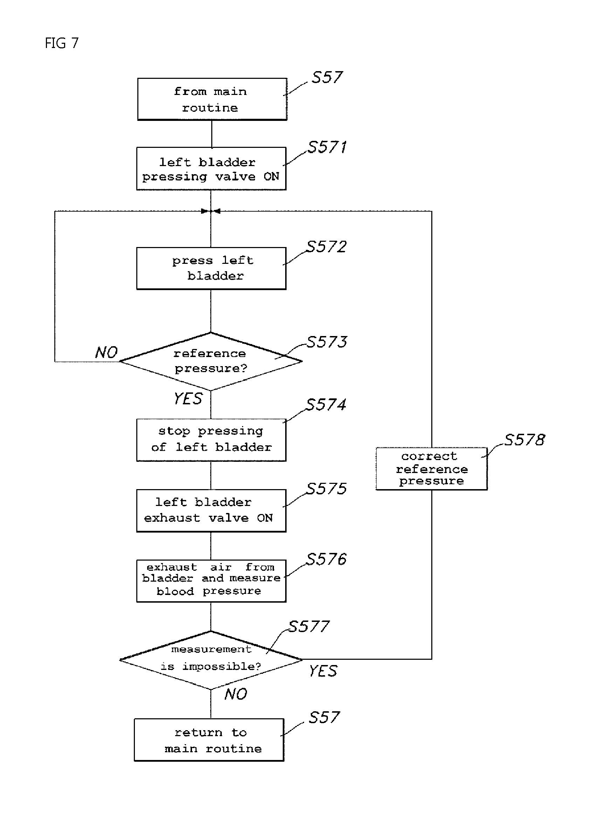

FIG. 7 is a flowchart showing the sub-routine to explain the operation to measure the blood pressure of the left and right arms shown in FIG. 6.

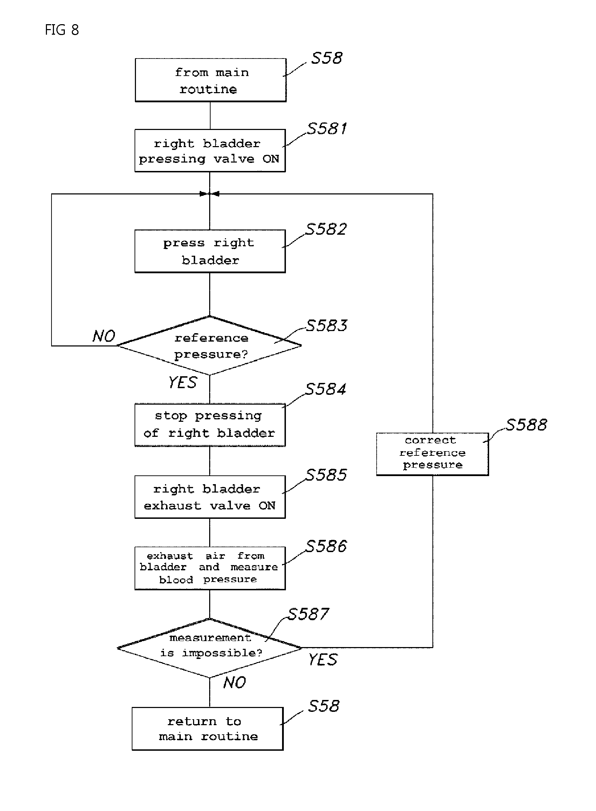

FIG. 8 is a flowchart showing the sub-routine to explain the operation to measure the blood pressure of the right arm shown in FIG. 6.

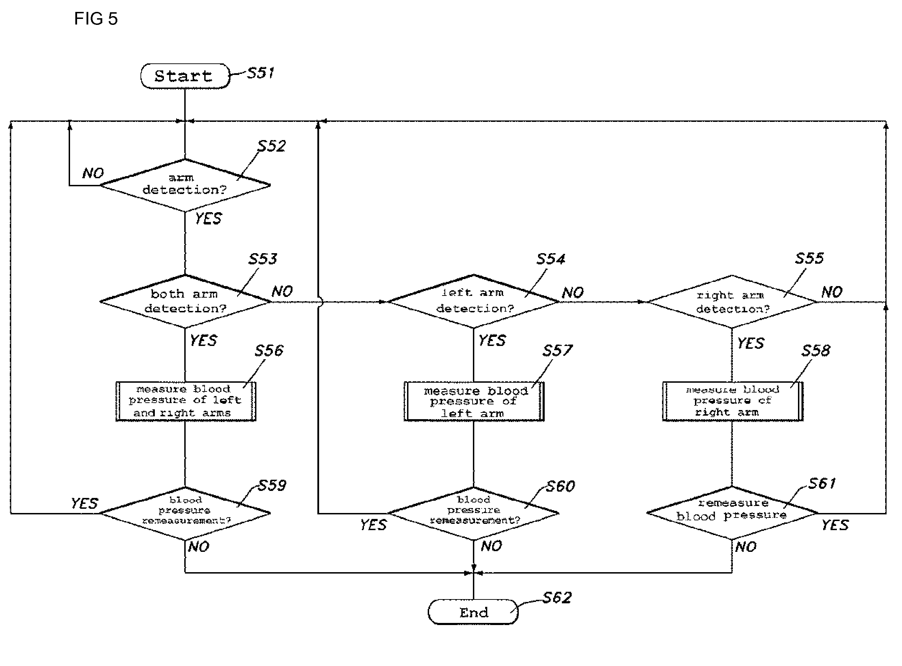

Hereinafter, the operation of the apparatus 100 for measuring the blood pressures of both arms will be described with reference to FIG. 5.

When the examinee inserts the lower arms and the wrists into the openings 111 and 211 of the left and right cuff units 110 and 210 to pass the lower arms and the wrists through the inner space parts of the left and right cuff units 110 and 210, and places the elbows on the elbow support members 120 and 220, and places lower arms and the wrists on the arm support members 140 and 240 in an inclination direction along the arm support members 140 and 240, the measurement portions of left and right upper arms of the examinee are placed in the left and right cuff units 111 and 211 at the same height.

If the operation is started, the CPU 300 performs step S52 to determine if the arms of the examinee are detected. The types of the arms of the examinee are determined by signals of the left and right detecting sensors 130 and 230.

If the arms are detected in step S52, the CPU 300 performs step S53 to determine if both arms are detected. If both arms are detected, the CPU 300 performs step S56 to enter the measurement mode of both of the left and right arms. If the both arms are not detected, the CPU 300 performs step S54 or step S55.

If the left arm is detected in step S54, the CPU 300 performs step S57 to enter the measurement mode of the left arm. If the left arm is not detected, the CPU 300 performs step S55. If the right arm is detected in step S55, the CPU 300 performs step S58 to enter the measurement mode of the right arm. If the right arm is not detected, the CPU 300 returns to step S52 and waits for the detection of the arms.

If the measurement of the blood pressures of the left and right arms is finished in step S56, the CPU 300 performs step S59 to determine if the blood pressure is remeasured. If the blood pressure is remeasured, the CPU 300 returns to step S52. If the blood pressure is not remeasured, the CPU 300 terminates the measurement of the blood pressures. If the measurement for the left arm is finished in step S57, the CPU 300 performs step S60 to determine if the blood pressure is remeasured. If the blood pressure is remeasured, the CPU 300 returns to step S52. If the blood pressure is not remeasured, the CPU 300 terminates the measurement of the blood pressure. If the measurement for the right arm is finished in step S58, the CPU 300 performs step S61 to determine if the blood pressure is remeasured. If the blood pressure is remeasured, the CPU 300 returns to step S52. If the blood pressure is not remeasured, the CPU 300 terminates the measurement of the blood pressure.

Hereinafter, the operation to measure the blood pressure will be described by using the sub-routine.

The following description will be made on the assumption that both arms are detected.

If both of left and right arms are detected in step S53, the CPU 300 performs the sub-routine of FIG. 6. The CPU 300 performs step S561 to control the left and right bladder pressing valve driving circuits 331 and 431 to turn on the left and right bladder pressing valves 333 and 433. In addition, the CPU 300 performs step S562 to control the air pump driving circuit 301 to drive the air pump 303, thereby pressing the left and right bladders 117 and 217 by injecting air into the left and right bladders 117 and 217.

The pressures of the left and right bladders 117 and 217 are continuously detected by the left and right pressure sensors 341 and 441 during the pressing process and sent to the CPU 300 through the left and right AD converters 343 and 443. The CPU 300 performs step S563 to determine if the detected pressures reach a reference pressure. If the detected pressures reach the reference pressure, the CPU 300 performs step S564 to stop pressing of the left and right bladders 117 and 217. If the pressures of the left and right bladders 117 and 217 do not reach the reference pressure in step S563, the CPU 300 returns to step S562 to continuously drive the air pump 303, thereby injecting air into the left and right bladders 117 and 217.

If the pressures of the left and right bladders 117 and 217 reach the reference pressure in step S563, the CPU 300 performs step S564 to stop pressing of the left and right bladders 117 and 217. Then, the CPU 300 performs step S565 to control the left and right bladder exhaust valve driving circuits 351 and 451, so that the left and right bladder exhaust valve 353 and 453 are turned on. Next, the CPU 300 performs step S566 to exhaust air of the left and right bladders 117 and 217 while measuring the blood pressure of the left and right arms.

The CPU 300 performs step S567 to determine if the measurement of the blood pressure is possible. If the pressures of the left and right bladders 117 and 217 are not higher than the blood pressure of the examinee by a predetermined value, the blood pressure of the examinee may not be measured. If the blood pressure is not measured for any one of the left and right arms of the examinee in step S567, the CPU 300 performs step S568 to correct the reference pressure of the left and right bladders 117 and 217. In addition, the CPU 300 returns to step S562 to press the left and right bladders 117 and 217 by injecting air into the left and right bladders 117 and 217.

If the measurement of the blood pressure is possible in step S567, so that the blood pressures of the left and right arms of the examinee are finished, the CPU 300 returns to the main routine.

As soon as the measurement of the blood pressures of both of the left and right arms is finished, the blood pressures of both of the left and right arms and the difference between the blood pressures of both arms are displayed. In addition, the blood pressures of both of the left and right arms and the difference between the blood pressures of both arms may be output through the printer 160.

The following description will be made on the assumption that the left arm is detected.

If the left arm is detected in step S54, the CPU 300 performs the sub-routine of FIG. 7. The CPU 300 performs step S571 to control the left bladder pressing valve driving circuit 331 so that the left bladder pressing valve 333 is turned on. The CPU 300 performs S572 to control the air pump driving circuit 301 so that the air pump 303 is driven to inject air into the left bladder 117 while pressing the left bladder 117.

The pressure of the left bladder 117 is continuously detected through the left bladder pressure sensor 341 during the pressing process, and transmitted to the CPU 300 through the left A/D converter 343. The CPU 300 performs step S573 to determine if the pressure of the left bladder 117 reaches the reference pressure. If the pressure of the left bladder 117 reaches the reference pressure, the CPU 300 performs step S574 to stop pressing of the left bladder 117. If the pressure of the left bladder 117 does not reach the reference pressure in step S573, the CPU 300 returns step S572 to continuously drive the air pump 303, thereby injecting air into the left bladder 117.

If the pressure of the left bladder 117 reaches the reference pressure in step S573, the CPU 300 performs step S574 to stop the pressing of the left bladder 117. The CPU 300 performs step S575 to control the left bladder exhaust valve driving circuit 351 so that the left bladder exhaust valve 353 is turned on. Next, the CPU 300 performs step S576 to exhaust air from the left bladder 117 while measuring the blood pressure.

The CPU 300 performs step S577 to determine if the measurement of the blood pressure is possible. If the pressure of the left bladder 117 is not higher than the blood pressure of the examinee by a predetermined value, the blood pressure of the examinee may not be measured.

If the blood pressure of the left arm of the examinee cannot be measured in step S577, the CPU 300 performs step S578 to correct the reference pressure of the left bladder 117. Next, the CPU 300 returns to step S572 to press the left bladder 117 by injecting air into the left bladder 117.

If the measurement of the blood pressure is possible in step S577 so that the measurement of the blood pressure in the left arm of the examinee is finished, the CPU 300 returns to the main routine.

As soon as the measurement of the blood pressure in the left arm of the examinee is finished, the blood pressure of the left arm is displayed on the display 150. In addition, the blood pressure of the left arm may be output through the printer 160.

The following description will be made on the assumption that the right arm is detected. If the CPU 300 detects the right arm in step S55, the CPU 300 performs the sub-routine of FIG. 8. The CPU 300 performs step S581 to control the right bladder pressing valve driving circuit 431 so that the right bladder pressing valve 433 is turned on. Next, the CPU 300 performs step S582 to control the air pump driving circuit 301 so that the air pump 303 is driven to inject air into the right bladder 217 by pressing the right bladder 217.

The pressure of the right bladder 217 is continuously detected by the right bladder pressure sensor 441 during the pressing process, and transmitted to the CPU 300 through the right A/D converter 443. The CPU 300 performs step S583 to determine if the pressure of the right bladder 217 reaches the reference pressure. If the pressure of the right bladder 217 reaches the reference pressure, the CPU 300 performs step S584 to stop the pressing of the right bladder 217. If the pressure of the right bladder 217 does not reach the reference pressure in step S584, the CPU 300 performs step S582 to continuously drive the air pump 303, thereby injecting air into the right bladder 217.

If the pressure of the right bladder 217 reaches the reference pressure in step S583, the CPU 300 performs step S584 to stop the pressing of the right bladder 217. Then, the CPU 300 performs step S585 to control the right bladder exhaust valve driving circuit 451 so that the right bladder exhaust valve 453 is turned on. Next, the CPU 300 performs step S586 to exhaust air from the right bladder 217 while measuring the blood pressure.

The CPU 300 performs step S587 to determine if the measurement of the blood pressure is possible. If the pressure of the right bladder 217 is not higher than the blood pressure of the examinee by a predetermined value, the blood pressure may not be measured. If the CPU 300 cannot measure the blood pressure of the right arm in step S587, the CPU performs step S588 to correct the reference pressure of the right bladder 217. In addition, the CPU 300 returns to step S582 to press the right bladder 217 by injecting air into the right bladder 217.

If the CPU 300 can measure the blood pressure in step S587 to terminate the measurement of the blood pressure in the right arm of the examinee, the CPU 300 returns to the main routine.

As soon as the measurement of the right-arm blood pressure of the examinee is finished, the blood pressure of the right arm is displayed on the display 150. In addition, the blood pressure of the right arm may be output through the printer 160.

According to the apparatus of the present invention, the blood pressures of both of the left and right arms are measured under the condition that the time spent to measure the blood pressures of both arms, the positions of both arms, and the pressures of the bladders are the same. For example, when the blood pressure of a left arm is very high, or when the blood pressure of the left arm cannot be measured, the same pressure is re-applied to a right arm as well as the left arm, thereby measuring the blood pressures of both blood pressures at the same pressure under the same condition.

Accordingly, the blood pressures of the left and right arms of the examinee are measured at the same pressure, the same condition, and the same time. Accordingly, the state related to the blood flow or the blood pressure state can be easily detected based on the difference between the blood pressures of the both arms.

Meanwhile, according to the apparatus of the present invention, an examinee can stop the blood pressure measurement by pressing an emergency switch during the blood pressure measurement.

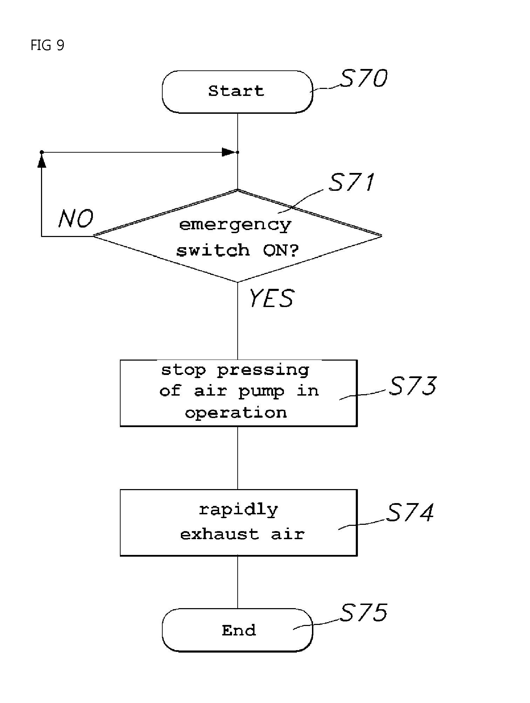

FIG. 9 is a flowchart to explain the operation of the emergency switch in the apparatus for measuring blood pressures of both arms according to the present invention.

The apparatus for measuring the blood pressures of both arms performs step S71 to continuously monitor that the emergency switch is pressed and turned on during the operation. If the emergency switch 172 is turned on, step S73 is performed to stop the air pump 303 in operation so that the pressing of the bladder is stopped. Next, step S74 is performed to control the rapid exhaust valve driving circuit 305, so that the rapid exhaust valve 306 is open to exhaust air from the bladder.

The emergency switch 172 is especially useful when the blood pressures of both of the left and right arms are measured. As shown in FIG. 11, the emergency switch 172 may include a foot switch 182 operated by a foot, and may include a bar switch 184 having the shape of a bar gripped by a hand.

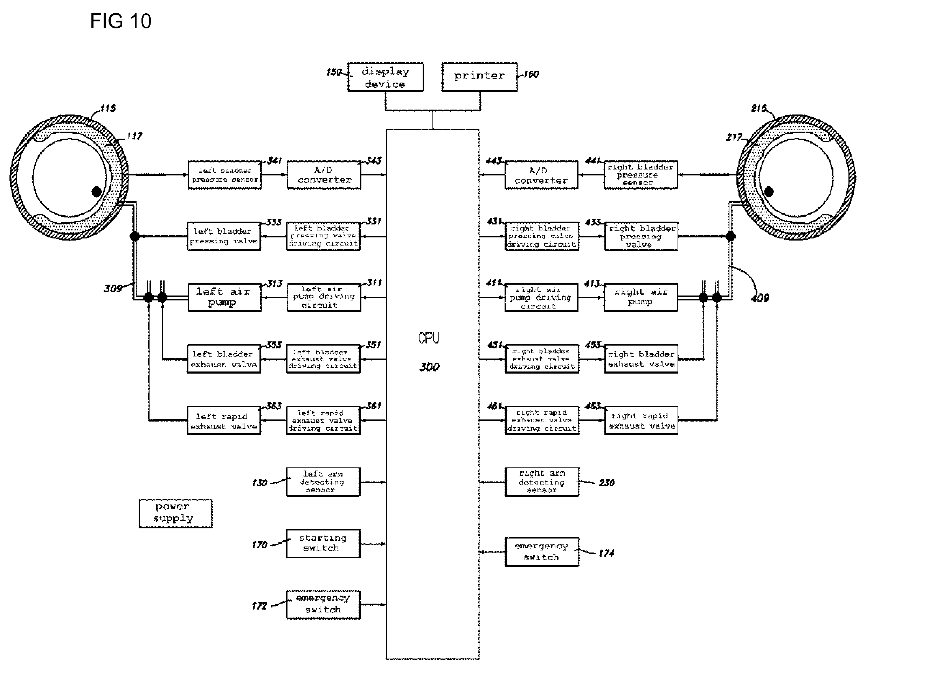

FIG. 10 is a block diagram showing the apparatus for measuring blood pressures of both arms of the examinee according to the embodiment of the present invention. When comparing the structure of FIG. 10 with the structure of FIG. 4, the air pump 303 is classified into the left air pump 303 to press the left bladder 117 by using air and the right air pump 413 to press the right bladder 217 by using air.

Accordingly, the bladder 117 constituting the left cuff unit 110 is connected to the left air supply pipe 309 from the left air pump 313, and the left bladder pressing valve 333, the left bladder exhaust valve 353, and the rapid exhaust valve 363 are attached to the left air supply pipe 309.

The left air pump 313 is driven according to the driving signal of the left air pump driving circuit 311, and the left bladder exhaust valve 353 is driven according to the driving signal of the left bladder exhaust valve driving circuit 351. The left rapid exhaust valve 363 attached to the left air supply pipe 309 is driven according to the driving signal of the left rapid exhaust valve driving circuit 361.

Accordingly, the bladder 217 constituting the right cuff unit 210 is connected to the right air supply pipe 409 from the right air pump 413, and the right bladder pressing valve 433, the right bladder exhaust valve 453, and the rapid exhaust valve 463 are attached to the right air supply pipe 409.

The right air pump 413 is driven according to the driving signal of the right air pump driving circuit 411, and the right bladder exhaust valve 453 is driven according to the driving signal of the right bladder exhaust valve driving circuit 451. The right rapid exhaust valve 463 attached to the right air supply pipe 409 is driven according to the driving signal of the right rapid exhaust valve driving circuit 361.

The apparatus shown in FIG. 10 operates based on the flowcharts shown in FIGS. 5 to 9.

As described above, according to the present invention, the examinee can simultaneously measure the blood pressures of both arms in a safety and stable posture without getting the arms out of the cuff units.

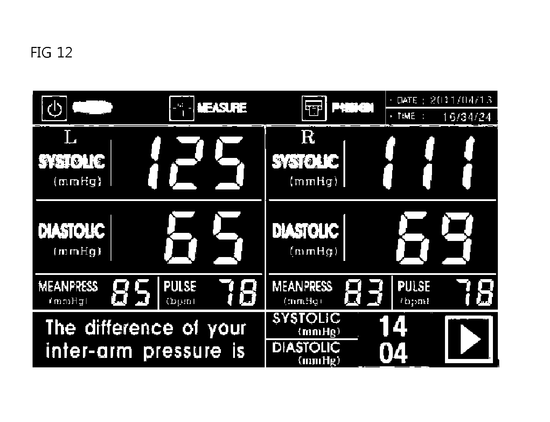

FIG. 12 is a view showing the measurement result of the blood pressures of the left and right arms displayed on a screen image in terms of the maximum blood pressure, the minimum blood pressure, and the difference between the maximum blood pressure and the minimum blood pressure.

Referring to FIG. 12, the maximum blood pressure, the minimum blood pressure, an average blood pressure, and a pulse of the left arm of the examinee are displayed on the left side of the screen image. In addition, on the right side of the screen image, the maximum blood pressure, the minimum blood pressure, an average blood pressure, and a pulse of the right arm of the examinee are displayed. On the lower portion of the screen image, the difference between the maximum blood pressures of the left and right arms, and the difference between the minimum blood pressures of the left and right arms are displayed.

### [Industrial Applicability]

The present invention provides an apparatus for automatically measuring blood pressures and a display to display the difference between the blood pressures, in which an examine can simultaneously and safely measure the blood pressures without getting the arms out of cuffs at the constant posture after placing both arms into the cuffs under the same condition.

## Claims

1. An apparatus for automatically and simultaneously measuring blood pressures of both arms, the apparatus comprising:
left and right cuff units receiving arms of an examinee therein and comprising bladders automatically wound around the arms in an operation; and
a control unit controlling the left and right cuff units,
wherein the left and right cuff units are symmetric to each other such that the blood pressures are measured at identical blood pressure measurement portions of the left and right arms of the examinee placed in internal space parts of the left and right cuff units, respectively, and front openings have an identical shape, rear openings have an identical shape, the internal space parts have an equal size, and bladders of the left and right cuff units have an equal volume such that the blood pressures are measured at the identical blood pressure measurement portions of the left and right arms of the examinee, which are placed in internal space parts of the left and right cuff units, respectively, under an identical condition, and
wherein the control unit simultaneously drives the left and right cuff units to simultaneously measure the blood pressures of the left and right arms, if the left and right arms of the examinee are placed in the internal space parts of the left and right cuff units.

2. The apparatus of claim 1, wherein the bladders of the left and right cuff units surround the identical blood pressure measurement portions of the left and right arms of the examinee.

3. The apparatus of claim 2, wherein the bladders of the left and right cuff units are in a plane-symmetric to each other to surround arteries of the both arms.

4. The apparatus of claim 3, wherein the blood pressures of the both arms are displayed on a screen image in terms of maximum blood pressures and minimums blood pressures of the left and right arms, a difference between the maximum blood pressures of the both arms, and a difference between the minimum blood pressures of the both arms.

5. The apparatus of claim 1, wherein the bladders of the left and right cuff units are in a plane-symmetric to each other to surround arteries of the both arms.

6. The apparatus of claim 5, wherein the blood pressures of the both arms are displayed on a screen image in terms of maximum blood pressures and minimums blood pressures of the left and right arms, a difference between the maximum blood pressures of the both arms, and a difference between the minimum blood pressures of the both arms.
